# EUROPÄISCHE PATENTSCHRIFT

(11) **EP 3 182 337 B1**
(45) Veröffentlichungstag und Bekanntmachung des Hinweises auf die Patenterteilung: **19.06.2019**
(21) Anmeldenummer: 16002611.8
(22) Anmeldetag: 08.12.2016
(51) Int. Cl.: G06K 19/07, G06K 19/077, A61B 90/98

(54) **RFID-TRANSPONDER FÜR EIN MEDIZINISCHES INSTRUMENT UND/ODER FÜR EIN ENDOSKOP, MEDIZINISCHES INSTRUMENT UND/ODER ENDOSKOP SOWIE MONTAGEVERFAHREN**
RFID TRANSPONDER FOR A MEDICAL INSTRUMENT AND/OR FOR ENDOSCOPE, MEDICAL INSTRUMENT AND/OR ENDOSCOPE AND METHOD OF INSTALLING SUCH A DEVICE
TRANSPONDEUR RFID POUR UN INSTRUMENT MÉDICAL ET/OU POUR UN ENDOSCOPE, INSTRUMENT MÉDICAL ET/OU ENDOSCOPE ET PROCÉDÉ DE MONTAGE

(30) Priorität: 16.12.2015 DE 102015016233
(43) Veröffentlichungstag der Anmeldung: 21.06.2017
(60) Teilanmeldung: 17020463.0
(73) Patentinhaber: Karl Storz SE & Co. KG, 78532 Tuttlingen (DE)
(72) Erfinder: KUBE, Raik, 22549 Hamburg (DE); CZUPALLA, Christian, 78532 Tuttlingen (DE)
(74) Vertreter: Fugmann, Winfried

(56) Entgegenhaltungen:
- WO-A1-2012/074378
- WO-A1-2015/036519
- US-A1- 2007 290 856

## Beschreibung

Die vorliegende Erfindung betrifft einen RFID-Transponder für ein medizinisches Instrument und/oder für ein Endoskop nach dem Oberbegriff von Anspruch 1, ein medizinisches Instrument bzw. ein Endoskop mit einem derartigen Transponder sowie ein Montageverfahren für ein medizinisches Instrument bzw. Endoskop mit einem RFID-Transponder.

Bei klinischen Abläufen werden eine Vielzahl medizinischer Instrumente gehandhabt. So müssen beispielsweise im Zusammenhang mit einer chirurgischen Operation die hierfür benötigten Instrumente bereitgestellt, überprüft und nach der Benutzung der Entsorgung oder Aufbereitung zugeführt werden. Hierfür hat es sich als nützlich erwiesen, die medizinischen Instrumente mit einer Markierung zu versehen, die eine Identifizierung und Nachverfolgung ermöglicht. Zur Erhöhung der Effizienz und Sicherheit der Abläufe ist dabei ein maschinelles Lesen der Markierung wünschenswert.

Es ist bekannt, medizinische Instrumente mit eindimensionalen Codes (Strichcode, Barcode) oder zweidimensionalen Codes, etwa Matrixcodes, zu versehen, die mit entsprechenden optischen Lesegeräten eingelesen werden können. Dieser Lesevorgang unterliegt jedoch einer Vielzahl von Einflüssen, die die Sicherheit des Einlesens verringern. So sind etwa mittels Laser auf chirurgische Instrumente aufgebrachte Barcodes oft schon nach etwa einem halben Jahr der Verwendung im OP-Einsatz verblichen und nur noch schlecht oder gar nicht mehr lesbar. Mechanisch aufgebrachte Codes führen zum Rostansatz und unterliegen einer ähnlichen Verschlechterung.

Weiter ist es bekannt, Funkfrequenz-Identifizierungs-Transponder, die auch als RFID-Transponder (Radio Frequency Identification) oder RFID-Tag bezeichnet werden, zur Markierung medizinischer, insbesondere chirurgischer Instrumente zu verwenden. Der RFID-Tag enthält einen Speicher, in dem Identifikationsdaten des Instruments wie etwa eine Seriennummer gespeichert sind, die mittels eines elektronischen Lesegeräts ausgelesen werden können. Bei vielen chirurgischen Instrumenten, insbesondere bei endoskopischen Instrumenten, ist der verfügbare Bauraum für die Integration eines RFID-Tags in das Instrument jedoch eng begrenzt. Ferner beträgt die Lesereichweite bei den RFID-Tags, die üblicherweise auf den HF (Hochfrequenz)-Bereich abgestimmt sind mit einer Betriebsfrequenz von 13,56 MHz und die eine miniaturisierte Bauform aufweisen, nur wenige Millimeter. Für eine sichere Datenübertragung ist es deshalb notwendig, den Lesekopf des Lesegeräts möglichst dicht am RFID-Tag zu platzieren. Die RFID-Tags sind daher in der Regel bei medizinischen bzw. chirurgischen Instrumenten von außen frei zugänglich und sichtbar, um ein entsprechend nahes Heranführen des Lesekopfs zu ermöglichen. Ein solcher oberflächlich angeordneter RFID-Transponder kann jedoch bei der Handhabung des Instruments mechanisch störend sein oder zumindest als störend empfunden werden sowie gegebenenfalls Oberflächenunebenheiten erzeugen, die die Reinigung des Instruments erschweren. Ferner ist eine solche Anordnung des RFID-Tags mit einem hohen fertigungstechnischen Aufwand verbunden.

Aus DE 197 23 442 A1 ist ein Endoskop mit einer im Endoskopinnenraum angeordneten Registriereinrichtung bekannt, die zumindest einen Umgebungsparameter erfasst, dem das Endoskop ausgesetzt wird, wobei die von der Registriereinrichtung registrierten Informationen von außen abfragbar sind. Die Registriereinrichtung kann zusammen mit den gespeicherten Daten eine eindeutige Identifikation des Endoskops übertragen.

Gemäß DE 200 12 237 U1 ist bei einem Endoskop ein Transponder in eine Ausnehmung der Außenfläche des Gehäuses des Endoskops schwimmend eingebettet. In DE 10 2011 052 501 A1 wird ein RFID-Tag zur Bestückung chirurgischer Instrumente vorgeschlagen, der eine Metallfassung umfasst sowie ein RFID-Element mit einer Antenne, die räumlich im Wesentlichen außerhalb der Metallfassung angeordnet ist. Gemäß US 2014/0210977 A1 ist ein Endoskopsystem zur drahtlosen Übermittlung von Energie und Daten ausgebildet, wobei ein Endoskop und ein mit dem Endoskop koppelbarer Kamerakopf jeweils einen. Transponder/Transceiver aufweisen. Beide Transponder/Transceiver sind zum drahtlosen Senden und Empfangen von Signalen voneinander eingerichtet.

Aus US 2004/0092991 A1 ist ein chirurgisches Werkzeugsystem mit einem Handstück und einem Schneidansatz bekannt, wobei ein RFID-Chip an einer Innenwand einer äußeren Nabe des Schneidansatzes angeordnet ist. Der RFID-Chip ist auf einem kleinen flexiblen Schaltkreis montiert, der eine Leiterbahn trägt, die eine Spule bildet. Im Handstück ist eine weitere Spule untergebracht, die zum Lesen und Schreiben von Daten in den RFID-Chip dient.

In EP 2 210 219 B1 ist ein RFID-Tag offenbart, der bei medizinischen Anwendungen verwendet werden kann und der hermetisch dicht und sterilisierbar ausgebildet ist. Der RFID-Tag umfasst eine Leiterplattenbaugruppe mit einer Leiterplatte, einem RFID-Schaltkreis, einer Antenne und einer metallischen Masseplatte, wobei die Leiterplatte eine erste Seite und eine zweite Seite aufweist, wobei die Leiterplatte auf ihrer ersten Seite die Antenne und auf ihrer zweiten Seite die Masseplatte trägt und wobei der RFID-Schaltkreis mit der Antenne und der Masseplatte gekoppelt ist. Der RFID-Tag umfasst weiterhin ein überformtes Gehäuse, welches die Leiterplattenbaugruppe umgibt und hermetisch abschließt, wobei das Gehäuse aus einem Material besteht, das sterilisierbar ist und das eine dielektrische Konstante zwischen annähernd 1 und 5 aufweist. Die Antenne kann eine gefaltete Konfiguration aufweisen mit einem zentralen Teil, der sich vom RFID-Schaltkreis zu einem Ende der Leiterplatte erstreckt, und mit einem Paar von entgegengesetzt gerichteten Armen.

Aus der gattungsfremden WO 2006/080615 A1 ist eine Öse bekannt, die eine Ösenbasis aus einem leitfähigen Material umfasst, die einen Ringteil, der eng an einem Objekt angebracht ist, und einen Befestigungsteil umfasst. Die Öse weist ferner ein RFID-Schaltkreismodul auf, das an der Ösenbasis montiert ist, um die Ösenbasis als Antenne zu nutzen. In der Ösenbasis ist ein Schlitz ausgebildet, und das RFID-Schaltkreismodul ist elektrisch mit den Teilen, die durch den Schlitz definiert werden, verbunden. Das ebenfalls gattungsfremde Dokument US 2010/0007501 A1 beschreibt einen Container, der zum Aufnehmen medizinischer Produkte benutzt werden kann und der einen kreisförmigen RFID-Tag aufweist.

Gemäß WO 2012/074378 A1 umfasst ein Transponder für eine Ohrmarke für Tiere einen Transponder-Chip und eine damit verbundene planare Antennenkonfiguration. Der Transponder ist auf einer scheibenförmigen Trägerfolie montiert. Die Antennenkonfiguration umfasst einen Dipol, der an beiden Enden eine Endkapazität aufweist, und zwei mäanderförmige Verkürzungswindungen. Der Transponder-Chip und die Antennenkonfiguration sind über einen Impedanzanpassungs-Schaltkreis miteinander verbunden.

Es ist eine Aufgabe der Erfindung, einen RFID-Transponder anzugeben, der zur Markierung eines medizinischen Instruments und/oder eines Endoskops geeignet ist, wobei die oben genannten Nachteile möglichst vermieden werden. Insbesondere soll der RFID-Transponder in ein medizinisches Instrument bzw. ein Endoskop auf einfache Weise integrierbar sein und möglichst eine höhere Lesereichweite und/oder eine geringere Winkelabhängigkeit beim Auslesen als bekannte, in medizinische Instrumente integrierte RFID-Transponder bieten. Weiter ist es eine Aufgabe der Erfindung, ein medizinisches Instrument bzw. ein Endoskop mit einem entsprechenden RFID-Transponder sowie ein Montageverfahren für ein medizinisches Instrument bzw. ein Endoskop mit einem RFID-Transponder anzugeben.

Diese Aufgabe wird durch einen RFID-Transponder gemäß Anspruch 1, durch ein medizinisches Instrument bzw. ein Endoskop gemäß Anspruch 12 sowie durch ein Montageverfahren gemäß Anspruch 14 gelöst.

Vorteilhafte Weiterbildungen der Erfindung ergeben sich aus den Unteransprüchen.

Ein erfindungsgemäßer RFID-Transponder, der auch als RFID-Tag bezeichnet wird, ist zur Markierung eines medizinischen Instruments und/oder eines Endoskops ausgebildet. Der Begriff "medizinisches Instrument" umfasst insbesondere chirurgische Instrumente, endoskopische Instrumente und medizinische Endoskope. Die vorliegende Erfindung ist jedoch nicht auf medizinische Instrumente bzw. medizinische Endoskope beschränkt, sondern bezieht sich gleichfalls auf Endoskope für nicht-medizinische Anwendungen. Der RFID-Transponder ist dazu ausgebildet, Daten, die zur Identifikation des betreffenden medizinischen Instruments bzw. des Endoskops dienen können, zu speichern, wobei die Daten in an sich bekannter Weise mittels eines elektronischen RFID-Lesegeräts berührungslos erfasst werden können.

Ein erfindungsgemäßer RFID-Transponder umfasst mindestens eine Leiterplatte, die eine Antenne trägt, die als mindestens eine auf die Leiterplatte aufgebrachte Leiterbahn ausgebildet ist. Die Leiterplatte trägt somit mindestens eine Leiterbahn, die als Antenne des RFID-Transponders geeignet ist und insbesondere eine entsprechende Länge und Anordnung aufweist. Die Leiterplatte ist vorzugsweise aus einem Material mit einer dielektrischen Konstante zwischen etwa 4 und 80 gefertigt. Die Leiterplatte kann flexibel ausgebildet sein. Weiter umfasst der erfindungsgemäße RFID-Transponder einen RFID-Schaltkreis, der mit der Antenne direkt oder indirekt, d.h. mit oder ohne Zwischenschaltung eines oder mehrerer weiterer elektrischer oder elektronischer Bauteile, gekoppelt ist. Der RFID-Schaltkreis ist dabei derart mit der Antenne gekoppelt, dass eine Übertragung der in einem Speicher des RFID-Schaltkreises gespeicherten Identifizierungsdaten zu einem externen Empfänger, etwa einem entsprechenden RFID-Lesegerät, möglich ist. Weiter kann ein Empfangen von Daten und/oder eine elektrische Energieversorgung des RFID-Schaltkreises über die Antenne möglich sein. Der Transponder kann weitere Bauteile aufweisen, etwa eine oder mehrere weitere Leiterplatten, eine eigene Energiequelle wie etwa eine Batterie und/oder ein Gehäuse.

Erfindungsgemäß ist die mindestens eine Leiterplatte im Wesentlichen ringförmig ausgebildet. Die mindestens eine Leiterplatte bildet somit einen geschlossenen oder auch einen unterbrochenen Ring, der beispielsweise kreisförmig, oval oder elliptisch sein kann. Vorzugsweise bildet die mindestens eine Leiterplatte näherungsweise einen Kreisring. Die Antenne wird durch die auf der im Wesentlichen ringförmigen Leiterplatte aufgebrachte mindestens eine Leiterbahn gebildet, wobei die Leiterbahn auf einer, bezogen auf eine Mittelachse oder eine Symmetrieachse der im Wesentlichen ringförmigen Leiterplatte, sich radial erstreckenden Fläche der Leiterplatte ausgebildet ist. Die sich radial erstreckende Fläche ist beispielsweise eine parallel zur oder in der Ringebene liegende Fläche, oder auch etwa eine Schraubenfläche. Die mindestens eine Leiterbahn ist insbesondere planar und auf einer parallel zur oder in der Ringebene liegenden Fläche der Leiterplatte ausgebildet. Vorzugsweise ist der RIFD-Transponder insgesamt entsprechend der Form der Leiterplatte ebenfalls im Wesentlichen ringförmig, beispielsweise oval, elliptisch oder kreisringförmig ausgebildet, gegebenenfalls einschließlich weiterer Bauteile, wie etwa weiterer Leiterplatten und/oder eines Gehäuses.

Dadurch, dass die mindestens eine Leiterplatte, die die Antenne trägt, im Wesentlichen ringförmig ausgebildet ist und somit der Transponder insgesamt ringförmig ausgebildet sein kann, wird eine raumsparende Unterbringung in einem medizinischen Instrument bzw. in einem Endoskop ermöglicht. Insbesondere ist aufgrund der ringförmigen Ausgestaltung der mindestens einen Leiterplatte bzw. des Transponders insgesamt eine von der Leiterplatte bzw. dem Transponder umschlossene oder weitgehend umschlossene zentrale Öffnung vorhanden, die bei der Integration in ein medizinisches Instrument bzw. ein Endoskop einen oder mehrere Arbeitskanäle und/oder ein optisches System aufnehmen kann. Weiter wird hierdurch eine besonders vorteilhafte Ausgestaltung der Antenne des RFID-Transponders ermöglicht. Insbesondere wird es dadurch, dass die mindestens eine Leiterplatte, die die Antenne trägt, im Wesentlichen ringförmig ausgebildet ist, ermöglicht, innerhalb des medizinischen Instruments bzw. des Endoskops eine Antenne unterzubringen, die auch für einen Leseabstand von beispielsweise mehreren Zentimetern geeignet ist. Eine grundlegende Umkonstruktion oder eine Vergrößerung des medizinischen Instruments bzw. des Endoskops ist hierfür nicht erforderlich.

Vorzugsweise ist der RFID-Transponder als passiver Transponder ausgebildet. Ein solcher passiver Transponder weist keine eigene Energiequelle auf und wird in an sich bekannter Weise insbesondere bei einem Lesevorgang vom Lesegerät mit der für die Datenübertragung notwendigen elektrischen Energie versorgt. Dies ermöglicht eine einfache und kostengünstige Ausgestaltung des RFID-Transponders, dessen Funktionsfähigkeit zudem nicht durch die Lebensdauer einer eigenen Energiequelle begrenzt ist.

Weiter vorzugsweise ist der RFID-Transponder für den UHF-Frequenzbereich ausgebildet. Die Frequenz für die Datenübertragung sowie gegebenenfalls die Energieübertragung liegt somit vorzugsweise im Bereich von 865 bis 868 MHz, insbesondere etwa bei 866,5 MHz. Hierdurch ist ein Leseabstand von mehreren Zentimetern, beispielsweise etwa 30 cm, erreichbar.

Gemäß einer bevorzugten Ausführungsform der Erfindung ist die mindestens eine Leiterplatte als einfach unterbrochener Ring ausgebildet, vorzugsweise als einfach unterbrochener Kreisring; die mindestens eine Leiterplatte kann aber auch etwa einen unterbrochenen ovalen oder elliptischen Ring bilden. Gemäß dieser Ausführungsform ist die Leiterplatte somit näherungsweise als Ring bzw. Kreisring ausgebildet, der einen beispielsweise radial gerichteten Schlitz aufweist. Der Schlitz nimmt nur einen geringen Teil des Umfangs der Leiterplatte ein, beispielsweise weniger als ein Viertel des Umfangs, insbesondere weniger als ein Zehntel des Umfangs, besonders bevorzugt weniger als ein Hundertstel des Umfangs. Vorzugsweise ist die Leiterplatte als ebene Leiterplatte ausgebildet. Die durch die Unterbrechung des Kreisrings gebildeten Enden müssen nicht miteinander fluchten, insbesondere kann die Leiterplatte etwa die Form eines Sprengrings haben. Weiter vorzugsweise ist der RFID-Transponder insgesamt ebenfalls als einfach unterbrochener Ring ausgebildet, vorzugsweise entsprechend der Form der Leiterplatte als einfach unterbrochener Kreisring, der beispielweise die Form eines Sprengrings haben kann. Die Enden des RFID-Transponders sind dabei durch einen Spalt getrennt, der nur einen geringen Teil des Umfangs des RFID-Transponders einnimmt, beispielsweise weniger als ein Viertel des Umfangs, insbesondere weniger als ein Zehntel des Umfangs, besonders bevorzugt weniger als ein Hundertstel des Umfangs; die Enden des RFID-Transponders können auch aneinander anliegen. Dadurch, dass die Leiterplatte bzw. der Transponder als unterbrochener Kreisring ausgebildet ist, wird auf einfache Weise eine Montage der Leiterplatte bzw. des Transponders in einer ringförmigen Nut ermöglicht, die beispielsweise auf eine Außen- oder Innenseite eines ring- oder rohrförmigen Bauteils eines medizinischen Instruments oder eines Endoskops, insbesondere auf einer Innenseite einer Okularmuschel eines Endoskops, angeordnet ist.

In bevorzugter Weise ist die Antenne als Dipol ausgebildet, in besonders bevorzugter Weise als Ring-Dipol, der auf beiden Seiten der Unterbrechung der näherungsweise kreisringförmigen Leiterplatte angeordnet ist. Dabei kann in einem Bereich der Leiterplatte, die bezogen auf die Mittel- oder Symmetrieachse des Kreisrings näherungsweise gegenüber der Unterbrechung angeordnet ist, die Kopplung der Antenne mit dem RFID-Schaltkreis erfolgen. Der Bereich, in dem die Kopplung der Antenne mit dem RFID-Schaltkreis erfolgt, wird auch als "Speisepunkt" bezeichnet. Dadurch, dass die Antenne als Dipol, insbesondere als Ring-Dipol, ausgebildet ist, wird eine besonders vorteilhafte Ausnutzung der für die Aufbringung der als Antenne dienenden mindestens einen Leiterbahn zur Verfügung stehenden Fläche der mindestens einen Leiterplatte ermöglicht.

Vorzugsweise ist die Antenne als zumindest abschnittsweise gefaltete Leiterbahn ausgebildet. Dadurch, dass die Leiterbahn, die die Antenne bildet, zumindest abschnittsweise gefaltet ist, wird es ermöglicht, eine Leiterbahn bzw. eine Antenne mit einer größeren Länge auf der Leiterplatte anzubringen, als der entlang des Umfangs des Rings gemessenen Länge der Leiterplatte entspricht. Hierdurch kann eine verbesserte Anpassung der Antenne an eine Betriebsfrequenz, etwa an den UHF-Bereich, und somit eine größere Reichweite ermöglicht werden.

Gemäß einer besonders bevorzugten Ausführungsform der Erfindung ist die Antenne als Morgain-Dipol ausgebildet, der vorzugsweise ringförmig gekrümmt ist. Ein solcher Morgain-Dipol kann durch zwei je zweifach gefaltete, sich entlang der ringförmigen Leiterplatte erstreckende, planare Leiterbahnen gebildet werden. Diese können insbesondere von einem Speisepunkt, der der Unterbrechung näherungsweise gegenüber liegt, ausgehen und sich jeweils bis nahe an die Unterbrechung erstrecken, bis nahe an den Speisepunkt zurückreichen und sich wiederum bis nahe an die Unterbrechung erstrecken. Die Ausbildung der Antenne als Kombination eines Ringdipols mit einem Morgain-Dipol ermöglicht eine Anpassung der Antenne an die Form der Leiterplatte, wenn diese als unterbrochener Ring ausgebildet ist, und zudem eine weiter verbesserte Anpassung der Antennenlänge an die Abmessungen der im Wesentlichen ringförmigen Leiterplatte, die bei medizinischen Instrumenten bzw. Endoskopen durch den zur Verfügung stehenden Einbauraum begrenzt sind. Insbesondere kann bei hierfür geeigneten Abmessungen, etwa bei einem vorgegebenen Innendurchmesser des im Wesentlichen ringförmigen RFID-Transponders von ca. 1 cm und einer Ringbreite von ca. 3 mm, die Antennenlänge derart gewählt werden, dass eine Resonanzfrequenz im UHF-Bereich ermöglicht wird. Hierdurch kann eine weitere Erhöhung des Leseabstands und/oder eine Anpassung an eine gewünschte Betriebsfrequenz erreicht werden.

Erfindungsgemäß ist der RFID-Schaltkreis über eine Koppelschleife mit der Antenne gekoppelt. Die Koppelschleife kann als weitere Leiterbahn auf der mindestens einen Leiterplatte ausgebildet sein, insbesondere als planare Leiterbahn. Der RFID-Schaltkreis kann beispielweise als integrierter Schaltkreis (Integrated Circuit, IC) ausgebildet und in die Koppelschleife integriert sein. Dadurch, dass der RFID-Schaltkreis über eine Koppelschleife mit der Antenne gekoppelt ist, wird eine verbesserte Impedanzanpassung und somit eine Erhöhung der Reichweite ermöglicht. Ferner kann durch geeignete Wahl der Länge der Koppelschleife ein Blindanteil der Impedanz (Reaktanz) des RFID-Schaltkreises kompensiert werden.

Gemäß einer besonders bevorzugten Ausführungsform weist die Leiterplatte eine erste und eine zweite Seite auf, die auch als Ober- und Unterseite oder als Vorder- und Rückseite bezeichnet werden können. Gemäß dieser Ausführungsform ist die Antenne bzw. die diese bildende mindestens eine Leiterbahn auf der ersten Seite bzw. der Oberseite und die Koppelschleife, die ebenso als Leiterbahn ausgebildet sein kann, auf der zweiten Seite bzw. der Unterseite der Leiterplatte angeordnet. Vorzugsweise weist die Leiterplatte auf der Ober- und der Unterseite jeweils ebene Oberflächen auf, die parallel zu einer Ringebene bzw. senkrecht zur Mittelachse der Leiterplatte stehen, und die Leiterbahnen, die die Antenne und die Koppelschleife bilden, sind planare Leiterbahnen. Weiterhin kann auch der RFID-Schaltkreis auf der Unterseite angeordnet und insbesondere in die Koppelschleife integriert sein. Auf diese Weise kann eine besonders platzsparende Anordnung geschaffen werden.

Vorzugsweise sind die Antenne und die Koppelschleife auf der ersten und der zweiten Seite der mindestens einen Leiterplatte im Wesentlichen einander gegenüberliegend angeordnet. In besonders bevorzugter Weise sind jeweils eine äußere und eine innere Leiterbahn der Antenne und der Koppelschleife zumindest bereichsweise, senkrecht auf die Fläche der mindestens einen Leiterplatte gesehen, deckungsgleich. Hierdurch wird eine besonders wirksame Kopplung der Koppelschleife und der Antenne ermöglicht.

Vorzugsweise ist der Abstand zwischen der Antenne und der Koppelschleife zur Kompensation eines Wirkanteils (Resistanz) der Impedanz des RFID-Schaltkreises angepasst. Der Abstand zwischen den die Antenne und die Koppelschleife bildenden Leiterbahnen, die jeweils im Wesentlichen planar ausgebildet sind, ist insbesondere dann, wenn diese auf der ersten und der zweiten Seite der mindestens einen Leiterplatte einander gegenüber liegen, durch die in axialer Richtung gemessene Dicke der Leiterplatte bestimmt. Durch eine entsprechende Wahl der Dicke der Leiterplatte ist somit eine verbesserte Impedanzanpassung und damit eine weitere Verbesserung der Sende- und Empfangseigenschaften des Transponders möglich.

Weiterhin ist es bevorzugt, dass die Koppelschleife in Umfangsrichtung gegenüber der Antenne versetzt, d.h. asymmetrisch zu dieser angeordnet ist. Insbesondere liegt die Koppelschleife, bezogen auf die Mittelachse der als unterbrochener Kreisring ausgebildeten mindestens einen Leiterplatte, der Unterbrechung nicht genau gegenüber, sondern ist gegenüber dieser auf eine Seite der Leiterplatte verschoben. Durch eine derartige Speisepunktverschiebung kann eine Kompensation eines Wirkanteils der Impedanz des RFID-Schaltkreises erzielt werden, so dass ebenfalls eine verbesserte Impedanzanpassung und damit eine Verbesserung der Sende- und Empfangseigenschaften des Transponders erreichbar ist.

Gemäß einer besonders bevorzugten Ausführungsform weist der Transponder ein hermetisch geschlossenes Gehäuse auf, das die mindestens eine Leiterplatte mit der Antenne sowie den RFID-Schaltkreis und gegebenenfalls die Koppelschleife sowie eventuelle weitere Bauteile des Transponders umschließt und das vorzugsweise entsprechend der mindestens einen Leiterplatte ebenfalls in Form eines geschlossenen oder unterbrochenen Kreisrings ausgebildet ist. Das Gehäuse weist vorzugsweise eine dielektrische Konstante im Bereich zwischen etwa 1 und etwa 5 auf, um vorteilhafte Sende- und Empfangseigenschaften des Transponders zu ermöglichen. Ferner kann das Gehäuse aus einem medizinisch zugelassenen Material hergestellt sein und gegen chemische, physikalische und thermische Einflüsse, die bei den üblichen Sterilisierungsverfahren auftreten, unempfindlich ausgebildet sein. Weiterhin ist es bevorzugt, dass die Bauteile des Transponders, insbesondere die auf der mindestens einen Leiterplatte angeordneten Komponenten, zumindest näherungsweise einen gleichen Wärmeausdehnungskoeffizienten aufweisen. In besonders bevorzugter Weise ist das Gehäuse sowie der Transponder insgesamt derart ausgebildet, dass dieser durch die beim Autoklavieren auftretenden Temperaturen und Drücke nicht beschädigt wird.

Ein erfindungsgemäßes medizinisches Instrument und/oder Endoskop weist einen wie oben beschrieben ausgebildeten RFID-Transponder auf, der insbesondere in einem proximalen (d.h. benutzernahen) Endbereich des medizinischen Instruments bzw. Endoskops in dieses integriert ist. Dadurch, dass das medizinische Instrument bzw. das Endoskop einen erfindungsgemäßen RFID-Transponder umfasst, wird auf einfache und sichere Weise eine Identifikation des medizinischen Instruments bzw. des Endoskops ermöglicht.

Vorzugsweise ist der RFID-Transponder in eine Okularmuschel eines Endoskops derart integriert, dass der RFID-Transponder einen Strahlengang des Endoskops im Wesentlichen ringförmig umgibt. Der Strahlengang des Endoskops erstreckt sich somit durch eine von der Leiterplatte bzw. dem Transponder umschlossene oder weitgehend umschlossene zentrale Öffnung des RFID-Transponders. Insbesondere umgibt die im Wesentlichen ringförmig, etwa als einfach unterbrochener Kreisring, ausgebildete Leiterplatte den Strahlengang des Endoskops und ist beispielsweise etwa symmetrisch zu einer optischen Achse des Endoskops angeordnet. Der Strahlengang des Endoskops wird durch das optische System des Endoskops definiert und umfasst auch den Lichtweg im Bereich des Fensters des Okulars. Der Transponder kann also beispielsweise derart angeordnet sein, dass er das Fenster des Okulars umgibt, d.h. radial außenseitig um dieses herum angeordnet ist. Insbesondere ist der RFID-Transponder im Inneren der Okularmuschel aufgenommen. Gemäß diesem Aspekt der Erfindung wird ausgenutzt, dass ein erfindungsgemäßer RFID-Transponder hinsichtlich Form, Abmessungen, Lesereichweite und Frequenzbereich besonders für die Integration in eine Okularmuschel eines Endoskops geeignet ist bzw. in einfacher Weise hierfür angepasst werden kann. Das Endoskop kann in an sich bekannter Weise weiterhin einen Schaft, ein optisches System sowie ggf. einen Endoskopkopf umfassen, wobei die Okularmuschel an einem proximalen Ende des Schafts bzw. am Endoskopkopf angeordnet ist. Der RFID-Transponder kann beispielsweise in eine innen umlaufende Nut der Okularmuschel oder in ein auf einer Innenseite der Okularmuschel angeordnetes Einlegeteil eingesetzt sein. Alternativ kann der RFID-Transponder in die Okularmuschel eingegossen sein, was bereits bei einer Herstellung der Okularmuschel durch Spritzgießen erfolgen kann. Der RFID-Transponder ist vorzugsweise von außen nicht sichtbar. Dadurch, dass der RFID-Transponder in die Okularmuschel integriert ist, die in der Regel aus einem nicht-metallischen Material besteht, insbesondere aus einem für medizinische Zwecke geeigneten Kunststoff, kann es auf einfache Weise erreicht werden, dass der RFID-Transponder nicht von metallischen Bauteilen umschlossen ist und zumindest genügend Abstand zu inneren metallischen Flächen des Endoskops aufweist, so dass die Signal- und Energieübertragung vom bzw. zum RFID-Transponder nicht wesentlich behindert ist. In entsprechender Weise kann der RFID-Transponder statt in eine Okularmuschel in ein nichtmetallisches Gehäuse eines Endoskopkopfs eines Endoskops integriert sein. Ebenso kann der RFID-Transponder in entsprechender Weise in ein aus einem nicht-metallischen Material bestehendes Bauteil eines medizinischen Instruments integriert sein.

Gemäß einem erfindungsgemäßen Verfahren zur Montage eines medizinischen Instruments und/oder Endoskops mit einem RFID-Transponder wird ein RFID-Transponder, der wie oben beschrieben als unterbrochener Ring, insbesondere als einfach unterbrochener Kreisring, ausgebildet ist, bereitgestellt. Mindestens ein Bauteil des medizinischen Instruments bzw. des Endoskops wird mit einer ringförmigen Nut mit für den RFID-Transponder ausreichenden Abmessungen hergestellt. Der RFID-Transponder wird sodann in die Nut eingesetzt, insbesondere nach Art eines Sprengrings. Vorzugsweise ist die Nut als auf einer Innenseite eines Bauteils umlaufende Nut ausgebildet. Zum Einsetzen werden die beiden Enden des RFID-Transponders gegen die elastische Rückstellkraft der Leiterplatte und des Gehäuses zusammengedrückt und in eine überlappende Anordnung gebracht; der RFID-Transponder wird sodann mit einem Teil seines Umfangs in die Nut eingefügt, wonach die übrigen Bereiche des Umfangs und die beiden Enden sich aufgrund der elastischen Rückstellkraft spreizen und in die Nut einrasten. Vorzugsweise ist die Nut auf einer Innenseite einer Okularmuschel eines Endoskops ausgebildet, und die Okularmuschel wird nach dem Einsetzen des RFID-Transponders auf einen proximalen Endbereich eines Gehäuses des Endoskops aufgesetzt und dort fixiert. Hierdurch kann auf einfache und sichere Weise ein RFID-Transponder in ein medizinisches Instrument bzw. in ein Endoskop integriert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 ein Ausführungsbeispiel eines erfindungsgemäßen RFID-Transponders in einer Schrägansicht der Oberseite, wobei das Gehäuse teilweise aufgeschnitten ist;
Fig. 2 den RFID-Transponder gemäß Fig. 1 ebenfalls in einer Schrägansicht der Oberseite, jedoch ohne Gehäuse;
Fig. 3 den RFID-Transponder gemäß Fig. 1 in einer Schrägansicht der Unterseite, wobei das Gehäuse teilweise aufgeschnitten ist;
Fig. 4 den RFID-Transponder gemäß Fig. 1 ebenfalls in einer Schrägansicht der Unterseite, jedoch ohne Gehäuse;
Fig. 5 den proximalen Endbereich eines erfindungsgemäßen Endoskops gemäß einem ersten Ausführungsbeispiel in einem Längsschnitt;
Fig. 6 den proximalen Endbereich eines erfindungsgemäßen Endoskops gemäß einem zweiten Ausführungsbeispiel in einem halbseitigen Längsschnitt;
Fig. 7 den proximalen Endbereich eines erfindungsgemäßen Endoskops gemäß einem dritten Ausführungsbeispiel in einem Längsschnitt.

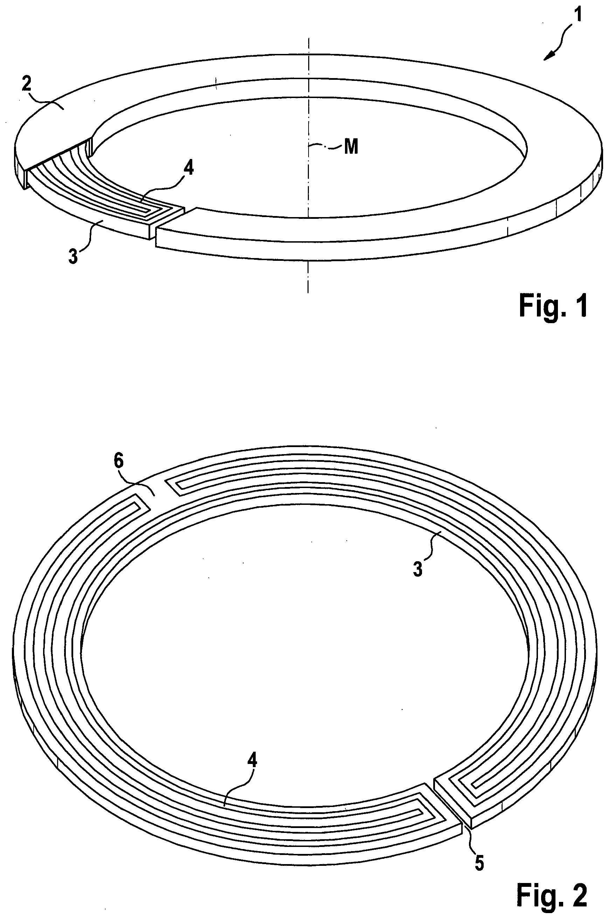

Wie in der in Fig. 1 gezeigten Schrägansicht eines gemäß einem Ausführungsbeispiel der Erfindung ausgebildeten RFID-Transponders 1 dargestellt ist, hat der RFID-Transponder 1 insgesamt die Form eines ebenen Kreisrings, der durch einen in radialer Richtung verlaufenden Spalt unterbrochen ist. Die Bezeichnungen "radial" und "axial" beziehen sich auf die in Fig. 1 angedeutete Mittelachse M des Kreisrings. Die Oberfläche des RFID-Transponders 1 wird allseitig durch ein Gehäuse 2 gebildet, das aus einem für medizinische Anwendungen zugelassenen Material besteht. Im Inneren des Gehäuses 2 ist, wie im aufgeschnittenen Bereich des Gehäuses 2 zu erkennen ist, eine Leiterplatte 3 aufgenommen, die auf ihrer Oberseite eine Leiterbahn 4 trägt, die die Antenne des RFID-Transponders 1 bildet.

Die Leiterplatte 3 ist in Fig. 2 ohne das Gehäuse 2 gezeigt. Wie in Fig. 2 dargestellt ist, hat die Leiterplatte 2 die Form eines ebenen Kreisrings, der durch den in radialer Richtung verlaufenden Spalt 5 unterbrochen ist. Die auf der Oberseite der Leiterplatte 3 verlaufende Leiterbahn 4 stellt einen ringförmig gekrümmten Morgain-Dipol dar. Ausgehend von einem Mittenbereich 6 der Leiterplatte 3, der bezogen auf die Mittelachse des Kreisrings gegenüber dem Spalt 5 angeordnet ist, verläuft die Leiterbahn 4 symmetrisch auf beiden Seiten des Mittenbereichs 6 nahe am inneren Rand der Leiterplatte 3 bis nahe an den Spalt 5, von dort nahe am äußeren Rand der Leiterplatte 3 zurück bis nahe an den Mittenbereich 6 und von dort etwa in der Mitte zwischen dem inneren und dem äußeren Rand wiederum bis nahe an den Spalt 5. Die Leiterbahn 4 stellt somit eine Kombination aus einem Ringdipol und einem verkürzten Morgain-Dipol dar und bildet auf beiden Seiten des Mittenbereichs 6 jeweils eineinhalb Windungen einer planaren Spule aus.

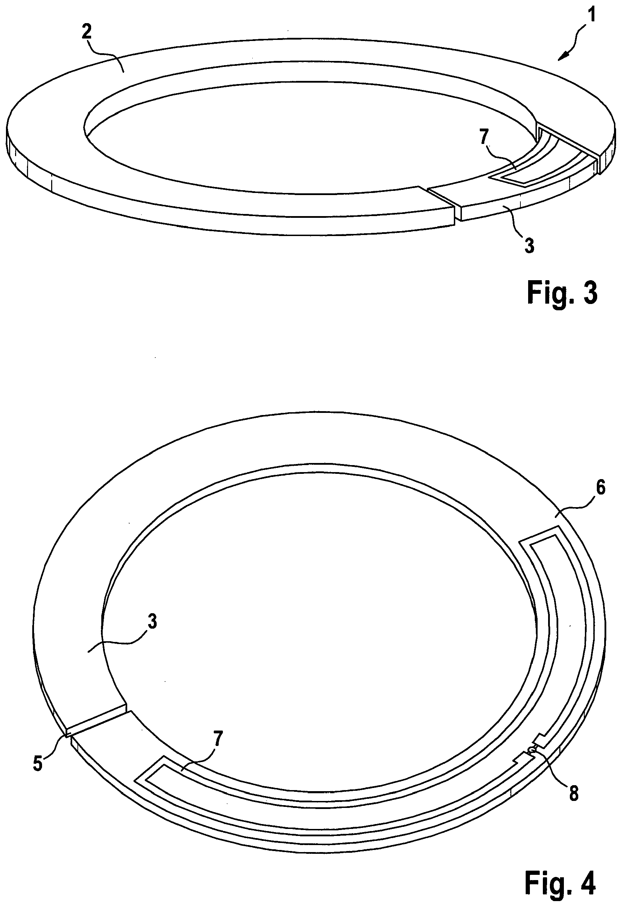

In den Figuren 3 und 4 ist in entsprechender Weise die Unterseite des RFID-Transponders 1 mit teilweise aufgeschnittenem Gehäuse 2 bzw. ohne das Gehäuse 2 gezeigt. Die Leiterplatte 3 trägt auf ihrer Unterseite eine weitere Leiterbahn 7, die eine Koppelschleife bildet. Zwischen die Enden der Koppelschleife ist ein RFID-Schaltkreis 8 eingeschaltet, der als integrierter Schaltkreis (IC) bzw. RFID-Chip ausgebildet ist. Die Leiterbahn 7 verläuft als Schleife entlang des inneren und des äußeren Rands der Leiterplatte 3, jedoch nur in einem Teilbereich der Länge der Leiterplatte 3. Im gezeigten Beispiel erstreckt sich die durch die Leiterbahn 7 gebildete Koppelschleife über nahezu die Hälfte der Länge der Leiterplatte 3 etwa von einem Bereich nahe dem Spalt 5 bis an den diesem gegenüberliegenden Mittenbereich 6 der Leiterplatte 3. Das Gehäuse 2 (s. Fig. 1 und 3) umschließt auch die Stirnseiten der Leiterplatte 3, die an den Spalt 5 angrenzen; der Spalt des RFID-Transponders 1 ist daher etwas schmaler als der Spalt 5 der Leiterplatte 3.

Im dargestellten Ausführungsbeispiel liegen die durch den Spalt 5 getrennten Enden der Leiterplatte 3 einander gegenüber. Abweichend hiervon können die Enden auch in axialer Richtung gegeneinander versetzt sein, wobei die Leiterplatte 3 die Form einer Helix haben kann. Das Gleiche gilt für den RFID-Transponder 1 insgesamt, der somit eine Form ähnlich einem Sprengring haben kann. Da die Leiterplatte 3 und das Gehäuse 2 zumindest in begrenztem Maße flexibel sind, kann zumindest durch Ausüben einer entsprechenden Kraft ein derartiger Versatz der Enden erreicht werden, was ein Eindrehen in eine Nut ähnlich einem Sprengring ermöglicht.

Die Länge der Antenne ist durch entsprechende Ausgestaltung der Leiterbahn 4 an eine Betriebsfrequenz des RFID-Transponders 1 im UHF-Bereich angepasst. Weiter ist durch die Ausgestaltung der Leiterbahnen 4, 7 sowie durch die Wahl der Dicke der Leiterplatte 3 die Impedanz der Antenne an die des RFID-Schaltkreises 8 angepasst. Die Impedanz üblicher RFID-Chips beträgt etwa 20 - 200 Ohm. Durch geeignete Wahl der Länge der Koppelschleife wird der Blindanteil der Impedanz des RFID-Schaltkreises 8 kompensiert. Eine Anpassung des Wirkanteils erfolgt hier durch den Versatz der Koppelschleife gegenüber dem Mittenbereich 6 in Umfangsrichtung der Leiterplatte 3 (Speisepunktverschiebung) sowie zudem durch eine geeignete Wahl der Dicke der Leiterplatte 3, die den Abstand zwischen der Ober- und der Unterseite der Leiterplatte 3 definiert und damit den axialen Abstand zwischen den Leiterbahnen 4, 7, d.h. zwischen der Antenne und der Koppelschleife.

In dem in den Figuren 1 bis 4 gezeigten Ausführungsbeispiel hat der RFID-Transponder 1 beispielsweise einen Innenradius von ca. 10,5 mm, einen Außenradius von ca. 11,9 mm und eine in axialer Richtung gemessene Dicke von ca. 1 mm oder weniger. Die Betriebsfrequenz des RFID-Transponders 1 liegt im UHF-Frequenzbereich (865 - 868 MHz). Aufgrund der beschriebenen Ausgestaltung der Antenne und der Koppelschleife kann eine Lesereichweite von etwa 30 cm bei einer geringen Winkelabhängigkeit erreicht werden.

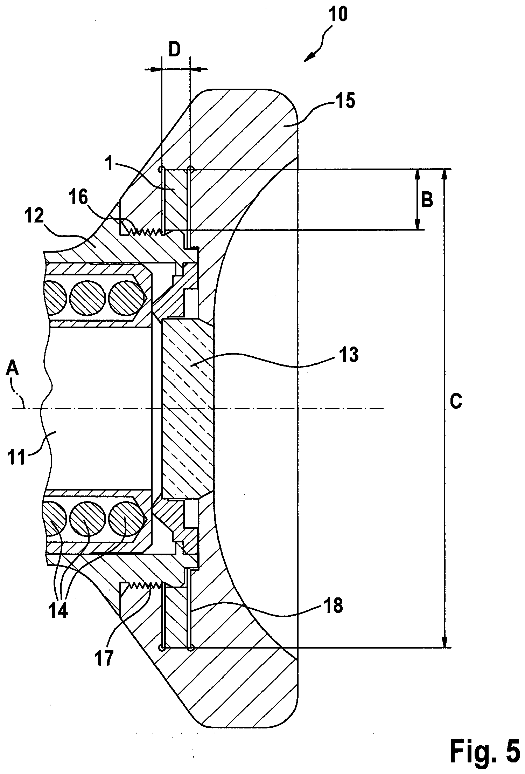

In Fig. 5 ist ein erstes Ausführungsbeispiel eines erfindungsgemäßen Endoskops 10 mit einem integrierten RFID-Transponder 1 gezeigt, wobei dieser gemäß dem in den Figuren 1 bis 4 dargestellten Ausführungsbeispiel ausgebildet ist. In Fig. 5 ist in einem Längsschnitt nur der proximale Endbereich des Endoskops 10 dargestellt. Der Innenraum 11 des Endoskops 10 ist von einem aus mehreren fluiddicht miteinander verbundenen Bauteilen gebildeten Endoskopgehäuse 12, das proximalseitig von einem Fenster 13 abgeschlossen wird, hermetisch dicht umschlossen. Weiter weist das Endoskop 10 in an sich bekannter Weise einen in Fig. 5 nicht dargestellten langerstreckten Schaft auf, der mit dem Endoskopgehäuse 12 fluiddicht verbunden ist und in dem ein Objektiv sowie ein etwa durch Relaislinsensysteme gebildeter Bildweiterleiter zur Weiterleitung des vom Objektiv erzeugten Bilds zum proximalen Endbereich angeordnet sind. Das optische System des Endoskops umfasst ferner eine Okularoptik zur Betrachtung des vom Bildweiterleiter weitergeleiteten Bilds. Innerhalb des Innenraums 11 sind Bauelemente der Okularoptik sowie ggf. des Bildweiterleiters aufgenommen (in Fig. 5 nicht dargestellt). Weiter sind im Innenraum 11 Trockenmittelelemente 14 angeordnet. In Fig. 5 ist ferner eine Längsachse A des Endoskops 10 angedeutet, die auch die Symmetrieachse des optischen Systems ist. Der RFID-Transponder 1, der gemäß Fig. 1 bis 4 als einfach unterbrochener Kreisring ausgebildet ist, umgibt ringförmig den Strahlengang des Endoskops 10, insbesondere das Fenster 13 des Okulars, und ist symmetrisch zur Längsachse A angeordnet.

Zur Erleichterung der visuellen Betrachtung des vom Bildweiterleiter weitergeleiteten Bilds durch das Fenster 13 sowie ggf. zum Anschluss einer endoskopischen Kamera weist das Endoskop 10 an seinem proximalen Ende eine Okularmuschel 15 auf. Diese ist auf ein im proximalen Endbereich des Endoskopgehäuses 12 gebildetes Außengewinde 16 aufgeschraubt und weist hierfür ein entsprechendes Innengewinde 17 auf. Proximalseitig des Innengewindes 17 weist die Okularmuschel 15 innenseitig eine umlaufende Nut 18 auf, in die der RFID-Transponder 1 eingesetzt ist. Die Abmessungen der Nut 18 entsprechen den äußeren Abmessungen des RFID-Transponders 1 oder sind geringfügig größer. Im dargestellten Ausführungsbeispiel hat die Nut 18 eine Tiefe B = 3 mm, einen am Grund der Nut gemessenen Durchmesser C = 24 mm und eine axiale Breite D = 1,2 mm. Die Mittelachse des im Wesentlichen ringförmigen RFID-Transponders 1 fällt näherungsweise mit der Längsachse A des Endoskops 10 zusammen. Die Integration des RFID-Transponders 1 in das Endoskop 10 verursacht keine Veränderung der Außenkontur des Endoskops 10, insbesondere keine Vergrößerung eines Abschnitts des Endoskops 10 und keine Oberflächenunebenheiten. Der RFID-Transponder 1 ist von außen nicht sichtbar, wobei aufgrund der beschriebenen Ausgestaltung mit einer Betriebsfrequenz im UHF-Bereich eine Lesereichweise von beispielsweise 30 cm erreichbar ist.

Bei der Herstellung des Endoskops 10 wird die Okularmuschel 16 beispielsweise aus einem festen Block aus Polyetheretherketon (PEEK) gedreht, wobei das Innengewinde 17 und die Nut 18 eingebracht werden. Der RFID-Transponder 1 wird sodann nach Art eines Sprengrings in die Nut 18 eingesetzt. Schließlich wird die Okularmuschel 15 mit ihrem Innengewinde 17 auf das Außengewinde 16 des Endoskopgehäuses 12 aufgeschraubt und beispielsweise durch Kleben fixiert.

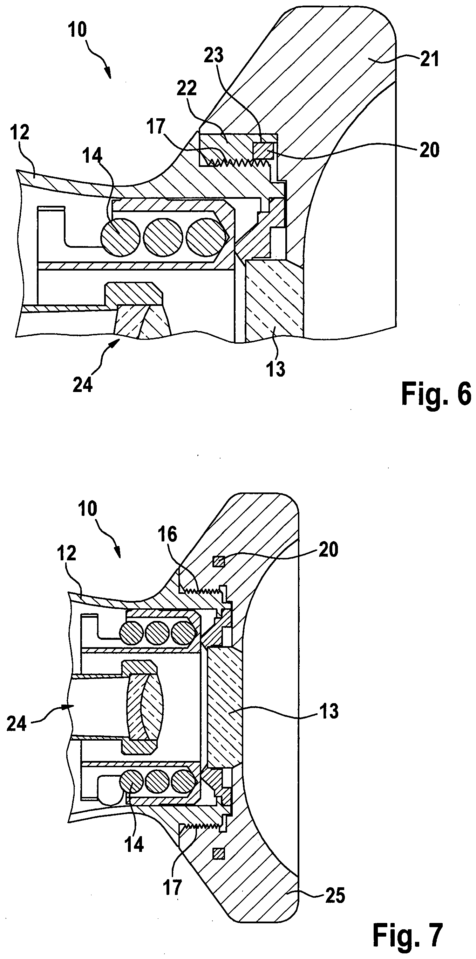

In den Figuren 6 und 7 sind beispielhaft zwei weitere Einbaumöglichkeiten eines erfindungsgemäßen RFID-Transponders 20 in den proximalen Endbereich eines Endoskops 10 bzw. in eine Okularmuschel 21, 25 dargestellt. Dabei kann der RFID-Transponder 20 wie der zuvor beschriebene RFID-Transponder 1 ausgebildet sein, kann aber auch als geschlossener Kreisring ohne Unterbrechung ausgebildet sein. Im letzteren Fall kann auch die Leiterplatte einen geschlossenen Kreisring darstellen, wobei die Antenne wie oben beschrieben (s. Fig. 1, 2) ausgebildet sein kann oder auch in an sich bekannter Weise durch eine planare, etwa spiralförmig umlaufende Spule auf der Oberseite der Leiterplatte gebildet werden kann. Ein RFID-Transponder, der einen geschlossenen Ring bildet und bei dem die Antenne durch eine etwa spiralförmig umlaufende Spule gebildet wird, kann aufgrund der hierbei zur Verfügung stehenden größeren Länge der entsprechenden Leiterbahn eine geringere radiale Breite haben. Zum Einbau gemäß Fig. 5 muss ein solcher RFID-Transponder ausreichend flexibel sein, um zum Einsetzen in die Nut 18 gefaltet werden zu können. Zum nachstehend beschriebenen Einbau gemäß Fig. 6 und 7 ist dies nicht erforderlich.

Gemäß dem in Fig. 6 dargestellten zweiten Ausführungsbeispiel eines erfindungsgemäßen Endoskops 10 weist die Okularmuschel 21 ein ringförmiges Einlegeteil 22 auf, das das Innengewinde 17 zum Aufschrauben auf das proximale Ende des Endoskopgehäuses 12 trägt. Das Einlegeteil 22 ist mit der Okularmuschel 21 beispielsweise durch Kleben fest verbunden. Am proximalen Ende des Einlegeteils 22 weist dieses innenseitig einen Einstich 23 auf, in den der RFID-Transponder 20 eingesetzt ist. Bei der Montage wird zunächst der ringförmige RFID-Transponder 20 in den Einstich 23 des Einlegeteils 22 eingesetzt und dieses sodann in die beispielsweise aus einem PEEK-Block gedrehte Okularmuschel 21 eingeschoben und mit dieser verbunden. Danach wird der Zusammenbau aus der Okularmuschel 21, dem Einlegeteil 22 und dem RFID-Transponder 20 auf das proximale Ende des Endoskopgehäuses 12 aufgeschraubt. In Fig. 6 ist neben den Trockenmittelelementen 14 auch das optische System 24 des Endoskops 10 angedeutet.

Bei dem in Fig. 7 dargestellten dritten Ausführungsbeispiel des Endoskops 10 ist der RFID-Transponder 20 in die Okularmuschel 25 eingegossen. Dieses kann beispielsweise direkt beim Spritzgießvorgang geschehen, mit dem die Okularmuschel 25 hergestellt wird. Die Okularmuschel 25 ist ähnlich wie zu Fig. 5 beschrieben mit einem Innengewinde 17 auf das Außengewinde 16 des Endoskopgehäuses 12 aufgeschraubt. Auch in Fig. 7 ist das optische System 24 des Endoskops 10 angedeutet. Im Übrigen ist bei dem zweiten bzw. dritten Ausführungsbeispiel das Endoskop 10 wie zu Fig. 5 beschrieben ausgebildet. Auch bei den Ausführungsbeispielen gemäß Fig. 6 und 7 ist der RFID-Transponder 20 in das Endoskop 10 im proximalen Endbereich integriert, ohne dass hierfür eine Veränderung der Außenkontur des Endoskops 10 notwendig wäre. Der RFID-Transponder 20 ist ebenfalls von außen nicht sichtbar, und es ist gleichfalls eine Lesereichweise von beispielsweise 30 cm erreichbar. Auch bei den in Fig. 6 und 7 gezeigten Ausführungsbeispielen umgibt der RFID-Transponder 20 den Strahlengang des Endoskops 10, der durch das optische System 24 und das Fenster 13 definiert ist, und ist symmetrisch zu diesem angeordnet.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: RFID-Transponder
- 2: Gehäuse
- 3: Leiterplatte
- 4: Leiterbahn
- 5: Spalt
- 6: Mittenbereich
- 7: Leiterbahn
- 8: RFID-Schaltkreis
- 10: Endoskop
- 11: Innenraum
- 12: Endoskopgehäuse
- 13: Fenster
- 14: Trockenmittelelement
- 15: Okularmuschel
- 16: Außengewinde
- 17: Innengewinde
- 18: Nut
- 20: RFID-Transponder
- 21: Okularmuschel
- 22: Einlegeteil
- 23: Einstich
- 24: Optisches System
- 25: Okularmuschel
- A: Längsachse
- B: Tiefe
- C: Durchmesser
- D: Breite
- M: Mittelachse

## Patentansprüche

1. RFID-Transponder für ein medizinisches Instrument und/oder für ein Endoskop (10), umfassend mindestens eine Leiterplatte (3), die eine Antenne trägt, die als mindestens eine Leiterbahn (4) der Leiterplatte (3) ausgebildet ist, und einen RFID-Schaltkreis (8), der mit der Antenne gekoppelt ist, wobei die mindestens eine Leiterplatte (3) im Wesentlichen ringförmig ausgebildet ist und wobei die mindestens eine Leiterbahn (4) auf einer sich radial erstreckenden Fläche der Leiterplatte (3) ausgebildet ist, **dadurch gekennzeichnet, dass** der RFID-Schaltkreis (8) über eine Koppelschleife mit der Antenne gekoppelt ist.

2. RFID-Transponder nach Anspruch 1, **dadurch gekennzeichnet, dass** der RFID-Transponder (1, 20) als passiver UHF-Transponder ausgebildet ist.

3. RFID-Transponder nach Anspruch 1 oder 2, **dadurch gekennzeichnet, dass** die Leiterplatte (3) als unterbrochener Ring, insbesondere als einfach unterbrochener Kreisring ausgebildet ist.

4. RFID-Transponder nach Anspruch 3, **dadurch gekennzeichnet, dass** der RFID-Transponder (1) als unterbrochener Ring, insbesondere als einfach unterbrochener Kreisring ausgebildet ist.

5. RFID-Transponder nach einem der vorhergehenden Ansprüche, **dadurch gekennzeichnet, dass** die Antenne als Dipol-Antenne, insbesondere als Ring-Dipol, ausgebildet ist.

6. RFID-Transponder nach einem der vorhergehenden Ansprüche, **dadurch gekennzeichnet, dass** die mindestens eine Leiterbahn (4) zumindest abschnittsweise gefaltet ist.

7. RFID-Transponder nach einem der vorhergehenden Ansprüche, **dadurch gekennzeichnet, dass** die Antenne als zumindest abschnittsweise ringförmig gekrümmter Morgain-Dipol ausgebildet ist.

8. RFID-Transponder nach einem der vorhergehenden Ansprüche, **dadurch gekennzeichnet, dass** die mindestens eine Leiterplatte (3) eine erste und eine zweite Seite aufweist, wobei die Antenne auf der ersten und die Koppelschleife auf der zweiten Seite angeordnet ist.

9. RFID-Transponder nach einem der vorhergehenden Ansprüche, **dadurch gekennzeichnet, dass** die Antenne und die Koppelschleife im Wesentlichen einander gegenüberliegend angeordnet sind.

10. RFID-Transponder nach einem der vorhergehenden Ansprüche, **dadurch gekennzeichnet, dass** zur Kompensation eines Wirkanteils der Impedanz des RFID-Schaltkreises (8) ein axialer Abstand zwischen der Antenne und der Koppelschleife angepasst ist.

11. RFID-Transponder nach einem der vorhergehenden Ansprüche, **dadurch gekennzeichnet, dass** zur Kompensation eines Wirkanteils der Impedanz des RFID-Schaltkreises (8) die Koppelschleife in Umfangsrichtung gegenüber der Antenne versetzt angeordnet ist.

12. Medizinisches Instrument und/oder Endoskop mit einem RFID-Transponder (1, 20) nach einem der vorhergehenden Ansprüche.

13. Endoskop nach Anspruch 12, **dadurch gekennzeichnet, dass** der RFID-Transponder (1, 20) in eine Okularmuschel (15, 21, 25) des Endoskops (10) integriert ist und einen Strahlengang des Endoskops (10) ringförmig umgibt.

14. Montageverfahren für ein medizinisches Instrument und/oder ein Endoskop (10) mit einem RFID-Transponder (20) gemäß einem der Ansprüche 1 bis 11 in Kombination mit Anspruch 4, wobei der RFID-Transponder (20) in eine ringförmige Nut (18) mindestens eines Bauteils des medizinischen Instruments bzw. Endoskops (10) eingesetzt wird.

## Claims

1. RFID transponder for a medical instrument and/or for an endoscope (10), comprising at least one printed circuit board (3) carrying an antenna embodied as at least one conductor track (4) of the printed circuit board (3) and an RFID circuit (8) coupled to the antenna, **wherein** the at least one printed circuit board (3) has a substantially ring-shaped embodiment and wherein the at least one conductor track (4) is embodied on a radially extending surface of the printed circuit board (3), **characterized in that** the RFID circuit (8) is coupled to the antenna by way of a coupling loop.

2. RFID transponder according to Claim 1, **characterized in that** the RFID transponder (1, 20) is embodied as a passive UHF transponder.

3. RFID transponder according to Claim 1 or 2, **characterized in that** the printed circuit board (3) is embodied as a ring with a break, in particular as a circular ring with one break.

4. RFID transponder according to Claim 3, **characterized in that** the RFID transponder (1) is embodied as a ring with a break, in particular as a circular ring with one break.

5. RFID transponder according to one of the preceding claims, **characterized in that** the antenna is embodied as a dipole antenna, in particular as a ring dipole.

6. RFID transponder according to one of the preceding claims, **characterized in that** the at least one conductor track (4) is folded, at least in sections.

7. RFID transponder according to one of the preceding claims, **characterized in that** the antenna is embodied as a Morgain dipole curved in a ring-shaped manner at least in sections.

8. RFID transponder according to one of the preceding claims, **characterized in that** the at least one printed circuit board (3) has a first side and a second side, wherein the antenna is arranged on the first side and the coupling loop is arranged on the second side.

9. RFID transponder according to one of the preceding claims, **characterized in that** the antenna and the coupling loop are arranged substantially opposite one another.

10. RFID transponder according to one of the preceding claims, **characterized in that** an axial distance between the antenna and the coupling loop is adapted for the purposes of compensating an active component of the impedance of the RFID circuit (8).

11. RFID transponder according to one of the preceding claims, **characterized in that** the coupling loop is arranged offset in relation to the antenna in the circumferential direction for the purposes of compensating an active component of the impedance of the RFID circuit (8).

12. Medical instrument and/or endoscope comprising an RFID transponder (1, 20) according to one of the preceding claims.

13. Endoscope according to Claim 12, **characterized in that** the RFID transponder (1, 20) is integrated into an eyepiece cup (15, 21, 25) of the endoscope (10) and surrounds a beam path of the endoscope (10) in a ring-shaped manner.

14. Assembly method for a medical instrument and/or an endoscope (10) comprising an RFID transponder (20) according to one of Claims 1 to 11 in combination with Claim 4, wherein the RFID transponder (20) is inserted into a ring-shaped groove (18) of at least one component of the medical instrument or endoscope (10) .

## Revendications

1. Transpondeur RFID pour un instrument médical et/ou pour un endoscope (10), comprenant au moins une carte de circuit imprimé (3) portant une antenne qui est réalisée sous la forme d'au moins une piste conductrice (4) de la carte de circuit imprimé (3), et un circuit RFID (8) qui est couplé à l'antenne,
dans lequel ladite au moins une carte de circuit imprimé (3) est réalisée de manière sensiblement annulaire et dans lequel ladite au moins une piste conductrice (4) est réalisée sur une surface s'étendant radialement de la carte de circuit imprimé (3), **caractérisé en ce que** le circuit RFID (8) est couplé à l'antenne par l'intermédiaire d'une boucle de couplage.

2. Transpondeur RFID selon la revendication 1, **caractérisé en ce que** le transpondeur RFID (1, 20) est réalisé sous la forme d'un transpondeur UHF passif.

3. Transpondeur RFID selon la revendication 1 ou 2, **caractérisé en ce que** la carte de circuit imprimé (3) est réalisée sous la forme d'un anneau interrompu, en particulier d'un anneau circulaire interrompu unique.

4. Transpondeur RFID selon la revendication 3, **caractérisé en ce que** le transpondeur RFID (1) est réalisé sous la forme d'un anneau interrompu, en particulier d'un anneau circulaire interrompu unique.

5. Transpondeur RFID selon l'une des revendications précédentes, **caractérisé en ce que** l'antenne est réalisée sous la forme d'une antenne dipôle, en particulier sous la forme d'un dipôle annulaire.

6. Transpondeur RFID selon l'une des revendications précédentes, **caractérisé en ce que** ladite au moins une piste conductrice (4) est repliée au moins par sections.

7. Transpondeur RFID selon l'une des revendications précédentes, **caractérisé en ce que** l'antenne est réalisée sous la forme d'un dipôle Morgain incurvé de manière annulaire au moins par sections.

8. Transpondeur RFID selon l'une des revendications précédentes, **caractérisé en ce que** ladite au moins une carte de circuit imprimé (3) présente des première et seconde faces, dans lequel l'antenne est disposée sur la première face et la boucle de couplage est disposée sur la seconde face.

9. Transpondeur RFID selon l'une des revendications précédentes, **caractérisé en ce que** l'antenne et la boucle de couplage sont disposées sensiblement l'une en face de l'autre.

10. Transpondeur RFID selon l'une des revendications précédentes, **caractérisé en ce que**, pour compenser une partie active de l'impédance du circuit RFID (8), une distance axiale est adaptée entre l'antenne et la boucle de couplage.

11. Transpondeur RFID selon l'une des revendications précédentes, **caractérisé en ce que**, pour compenser une partie active de l'impédance du circuit RFID (8), la boucle de couplage est décalée dans la direction de la circonférence par rapport à l'antenne.

12. Instrument médical et/ou endoscope comportant un transpondeur RFID (1, 20) selon l'une des revendications précédentes.

13. Endoscope selon la revendication 12, **caractérisé en ce que** le transpondeur RFID (1, 20) est intégré dans une coque d'oculaire (15, 21, 25) de l'endoscope (10) et entoure de manière annulaire un chemin lumineux de l'endoscope (10).

14. Procédé de montage d'un instrument médical et/ou d'un endoscope (10) comportant un transpondeur RFID (20) selon l'une des revendications 1 à 11 en association avec la revendication 4, dans lequel le transpondeur RFID (20) est inséré dans une rainure annulaire (18) d'au moins un composant de l'instrument médical ou de l'endoscope (10).
